(19) Europäisches Patentamt / European Patent Office / Office européen des brevets

(11) **EP 2 729 200 B1**

(12) **EUROPEAN PATENT SPECIFICATION**

(45) Date of publication and mention
of the grant of the patent:
**19.10.2016 Bulletin 2016/42**

(21) Application number: **11734051.3**

(22) Date of filing: **06.07.2011**

(51) Int Cl.:
***A61M 5/145*** *(2006.01)*      ***A61M 5/168*** *(2006.01)*

(86) International application number:
**PCT/EP2011/061441**

(87) International publication number:
**WO 2013/004307 (10.01.2013 Gazette 2013/02)**

(54) **AUTOMATIC INJECTION DEVICE COMPRISING TWO OCCLUSION SENSORS**

AUTOMATISCHES INJEKTIONGERÄT MIT ZWEI OKKLUSIONSDETEKTOREN

INJECTEUR AUTOMATIQUE COMPRENANT DEUX SENSEURS D'OCCLUSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.05.2014 Bulletin 2014/20**

(73) Proprietors:
• **F.Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Roche Diabetes Care GmbH
68305 Mannheim (DE)**
Designated Contracting States:
**DE**

(72) Inventor: **HÄNGGI, Roger
4208 Nunningen (CH)**

(74) Representative: **Rentsch Partner AG
Rechtsanwälte und Patentanwälte
Fraumünsterstrasse 9
Postfach 2441
8022 Zürich (CH)**

(56) References cited:
**EP-A1- 1 529 546      WO-A1-2008/106108
WO-A1-2009/053032      US-A- 5 989 222
US-A1- 2005 234 382**

**Description**

Field of the Invention

The present invention relates to an injection device for injecting automatically a medicament, and a method for detecting an occlusion in the injection device. Specifically, the present invention relates to an automatic injection device and a method for detecting an occlusion in the injection device using a force measurement unit for measuring an injection force. It is generally insulin that is injected into the body of a patient, although other medicaments to be injected over a long period of time can also be injected by the device, e.g. analgesics, pain killers or cancer drugs.

Background of the Invention

Automatically operating injection devices inject a predefined volume of a medicament into the body of a patient at predefined time intervals or delivery rates, respectively, in a continuous or quasi-continuous way. Typically, the delivery rate varies according to an individual schedule, e.g. according to a preprogrammed circadian profile. Some devices allow the additional injection of larger drug boli on demand. This volume is withdrawn from a reservoir, generally an exchangeable ampoule, via a pump mechanism and is injected through an injection needle placed in the patient's body. Automatically operating injection devices may be designed according to the syringe-driver principle. For carrying out an injection, a plunger that is received in a cartridge or ampoule is linearly displaced in a controlled way via a typically motor driven pump mechanism with a piston rod that contacts and pushes the piston during operation. If an occlusion is present in such a system, the pressure in the injection system increases, since there is no drop in pressure provided by injections. As a result, a force that is to be applied in the pump mechanism increases over several unsuccessfully performed injections. Thus, a force measurement makes it possible to ascertain whether or not there is an occlusion. If an occlusion occurs, the patient is no longer supplied with a necessary medicament, thus causing potentially severe medical complications. Moreover, since the pump unit operates automatically at predefined time intervals, the pressure in the ampoule and in the feed lines to the patient's body increases, which could cause damage to the injection device. A still greater problem is that, with increasing pressure, the occlusion may eventually break up abruptly and the patient may then receive too large a quantity of the medicament. With a measurement unit that determines an injection force necessary for discharging the medicament, it is possible to ascertain whether an occlusion is present.

US 6,659,980, for example, describes such an injection device in which the volume to be injected is discharged by a piston that can be advanced by a spindle driven by an electric motor. In order to detect an occlusion during injection, US 6,659,980 teaches a first method in which a maximum force threshold is predefined and, if it is exceeded, an occlusion alarm is triggered. In a second method, a force increase in a plurality of force values for discharging the injection volume is recorded. If no occlusion is present, then, according to the teachings of US 6,659,980, no increase in force is detectable over a period covering a plurality of injections; only when an occlusion occurs is there an increase over a plurality of measurements. To determine the increase in force, fifteen force values, each determined at identical time intervals, are evaluated in US 6,659,980.

WO 2009/53032 discloses an automatically operating injection device with a measurement unit for measuring and storing force values associated with injections of a medicament. According to WO 2009/53032 an evaluation unit determines an injection occlusion from the measurement values, whereby a switching unit modifies automatically the time interval between individual force measurements depending on evaluation results provided by the evaluation unit. WO 2009/53032 teaches that the time interval between the basal releases is maintained constant, e.g. three minutes, independently of the time intervals for recording the force values. Initially, the force measurements are taken every three minutes in sync with the basal releases and stored over 30 minutes, and only if the evaluation unit determines based on these force measurements a potential occlusion, there is an extension of the time interval for measuring the force values. However, for very low infusion rates, the time interval between the single basal releases has to be increased for typical devices due to a limited injection volume resolution. The time interval between basal releases may, e.g., be extended to 30 minutes for a smallest basal delivery rate of 0.02 IU/h (International Units per hour). Here, the interval between basal releases is longer than the time interval for measuring the force values, resulting in multiple substantially identical measurements between consecutive injections, rather than measurements being in sync with the injections. As a consequence, an occlusion is not detected in this situation.

The US 5989222 discloses an infusion pump with a pumping cassette, the pumping cassette having a first proximal and a second distal occlusion sensor in form of a proximal and a distal pressure sensor. In order adopt for different infusion flow rates, the US 5989222 teaches varying of the sensor sampling. The EP 1529546 A1 discloses an infusion device with a single occlusion detector that is realized by a force sensor. Signals provided by the force sensor are processed with a variable sampling time window. The US 2005234382 A1 discloses a syringe-type infusion device with a single occlusion detector that is realized by a force sensor. For detecting occlusions US 2005234382 A1 teaches determining the derivative of the force applied on a syringe plunger as a function of plunger displacement and comparing the derivative with a threshold value. The WO 2008106108 A1 discloses an automatic pump relay system with controllers coupled to drivers for sequentially discharging fluid from a plurality of medication containers into a coupler. The internal fluid pressure of the coupler is determined by a pressure sensing device or devices that provide signals to the controllers that, based at least on the sensor signals, cause the drivers to operate in reverse and forward directions.

In addition, some infusion systems provide a check valve or pressure valve between the ampule and the feed line that only opens at a certain opening pressure. After insertion of an ampule into the injection device, the opening pressure has to be built up in the fluidic system by advancing the piston before the feed line can be filled with medicine in a so-called priming phase and injection can start. Once, built up, the fluidic pressure in the ampule is maintained substantially at the opening pressure. Until the opening pressure is built up, the force values, as determined by the measurement unit, continuously increase in a similar way as in case of an occlusion during regular operation. Therefore, an occlusion detection as disclosed in WO 2009/53032 needs to be deactivated for the time before the actual injections starts.

In some situations, however, an ampule with connected valve and feed line is removed from the device during operation for some time and subsequently re-inserted. When removing the ampule, the opening pressure of the valve, present before, is released. After re-insertion of the ampule, the opening pressure has to be built-up again. Since the device is in a regular operation mode and the occlusion detection is activated, the force increase while building-up the opening pressure may result in a false occlusion alarm.

When inserting an ampule into the device, the ampule is typically fully filled with the plunger being in an end position. This however, is not necessarily the case, for example, if an ampule is filled by a device user only partly, or if readily filled ampules of different filling volumes are commercially available. This is also the case, if an ampule is removed from the device and subsequently re-inserted as described above.

When inserting a new cartridge, a piston rod of the drive mechanism may first be moved forward in a delivery direction, until it contacts the piston of the ampule and building-up the opening pressure of the pressure valve and priming can start. This phase of device operation is referred to as "sniffing phase". During the sniffing phase, the occlusion detection is favourably deactivated. If, however, an ampule is removed during operation and subsequently reinserted, building up the opening pressure and subsequent medicine delivery start immediately without sniffing phase and with the occlusion detection being activated.

Summary of the Invention

It is an object of this invention to provide an injection device for injecting automatically a medicament, and a method for detecting an occlusion in the injection device, which device and method do not have at least some of the disadvantages of the prior art. In particular, it is an object of the present invention to provide an automatically operating injection device and an occlusion detection method which are more flexible and efficient with regards to different basal delivery rates and delivery rate intervals, particularly with regards to low basal delivery rates.

According to the present invention, these objects are achieved through the features of the independent claims. In addition, further advantageous embodiments follow from the dependent claims and the description.

The above-mentioned objects are particularly achieved in that an injection device for injecting automatically a medicament, which comprises a force measurement unit configured to measure an injection force, further comprises a first occlusion detector configured to take force measurements at a first measurement rate, and to generate a first occlusion indicating signal based on a set of force measurements taken at the first measurement rate; a second occlusion detector configured to take force measurements at a second measurement rate, lower than the first measurement rate, and to generate a second occlusion indicating signal based on a set of force measurements taken at the second measurement rate; and an alarm generator configured to generate an occlusion alarm signal in cases where the first occlusion indicating signal is generated by the first occlusion detector and/or (logical "or") the second occlusion indicating signal is generated by the second occlusion detector, i.e. for cases where either one or both of the occlusion detectors indicate a detected occlusion.

Providing the injection device with two occlusion detectors which operate at different initial measurement rates has the advantage that occlusions can be detected more reliably over a broader range of delivery rates. Specifically, it becomes possible to detect occlusions not only at high delivery rates, e.g. an injection every three minutes, but also at much lower delivery rates, e.g. an injection every thirty minutes, depending on how the measurement rates are set.

Preferably, the injection device is configured to inject the medicament at a variable delivery rate, the first measurement rate corresponds to a defined upper delivery rate limit, and the second measurement rate corresponds to a defined lower delivery rate limit, lower than the defined upper delivery rate limit.

Preferably, the first occlusion detector is configured to generate the first occlusion indicating signal based on a set of force measurements, taken at the first measurement rate during a first measurement period; and the second occlusion detector is configured to generate the second occlusion indicating signal based on a set of force measurements, taken at the second measurement rate during a second measurement period longer than the first measurement period.

In an embodiment, the first occlusion detector is configured to generate the first occlusion indicating signal based on a first calculated product of a first vector of weighting factors and the set of force measurements, taken at the first measurement rate during the first measurement period; and the second occlusion detector is configured to generate the second occlusion indicating signal based on a second calculated product of a second vector of weighting factors and the set of force measurements, taken at the second measurement rate during the second measurement period longer than the first measurement period. For example, the first occlusion detector is configured to halve the first measurement rate in case the first calculated product is within a first defined threshold range; and the second occlusion detector is configured to halve the second measurement rate in case the second calculated product is within a second defined threshold range.

In a further embodiment, the first occlusion detector is configured to extend the first measurement period by doubling its duration in case the first calculated product is within a first defined threshold range; and the second occlusion detector is configured to extend the second measurement period by doubling its duration in case the second calculated product is within a second defined threshold range. Preferably, the first occlusion detector is configured to include in the extended first measurement period force measurements, taken at the first measurement rate before extending the first measurement period; and the second occlusion detector is configured to include in the extended second measurement period force measurements, taken at the second measurement rate before extending the second measurement period.

In an embodiment, the first occlusion detector is configured to generate the first occlusion indicating signal in case the first calculated product exceeds a first upper threshold value; and the second occlusion detector is configured to generate the second occlusion indicating signal in case the second calculated product exceeds a second upper threshold value.

In an additional embodiment, the injection device comprises an occlusion detection system configured to deduct from the force measurements a force offset value, and to detect the occlusions based on the force measurements having the force offset value deducted therefrom.

In addition, the above-mentioned objects are particularly achieved in that for detecting an occlusion in an injection device for injecting automatically a medicament, an injection force is measured; force measurements are taken at a first measurement rate by a first occlusion detector, and a first occlusion indicating signal based is generated based on a set of force measurements taken at the first measurement rate; force measurements are taken at a second measurement rate by a second occlusion detector and a second occlusion indicating signal is generated based on a set of force measurements taken at the second measurement rate; and an occlusion alarm signal is generated in cases where the first occlusion indicating signal is generated by the first occlusion detector and/or (logical "or") the second occlusion indicating signal is generated by the second occlusion detector.

Brief Description of the Drawings

The present invention will be explained in more detail, by way of example, with reference to the drawings in which:

Figure 1: shows an exemplary cross section of an injection device for injecting automatically a medicament into a body of a patient.

Figure 2: shows a block diagram illustrating schematically an automatically operating injection device with an occlusion detection system having a force measurement unit, two independent occlusion detectors, and an alarm generator.

Figure 3: shows a block diagram illustrating schematically an occlusion detector for an occlusion detection system of an automatically operating injection device.

Figure 4: shows a flow diagram illustrating an exemplary sequence of steps for detecting an occlusion in an automatically operating injection device.

Figure 5: shows graphs illustrating an example of occlusion detection including the temporal course of force measurements, and different dot products generated by occlusion detectors from force measurements taken at different measurement rates.

Detailed Description of the Preferred Embodiments

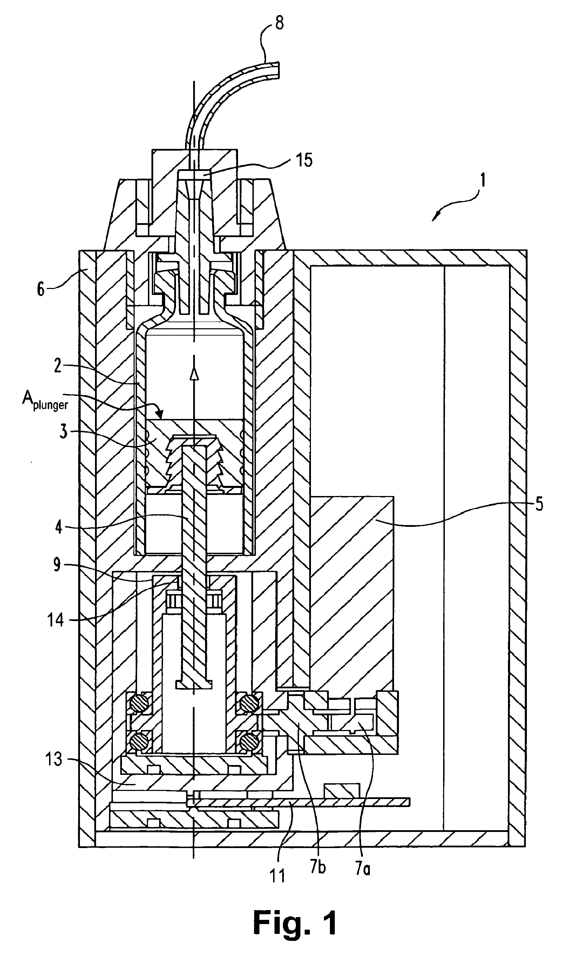

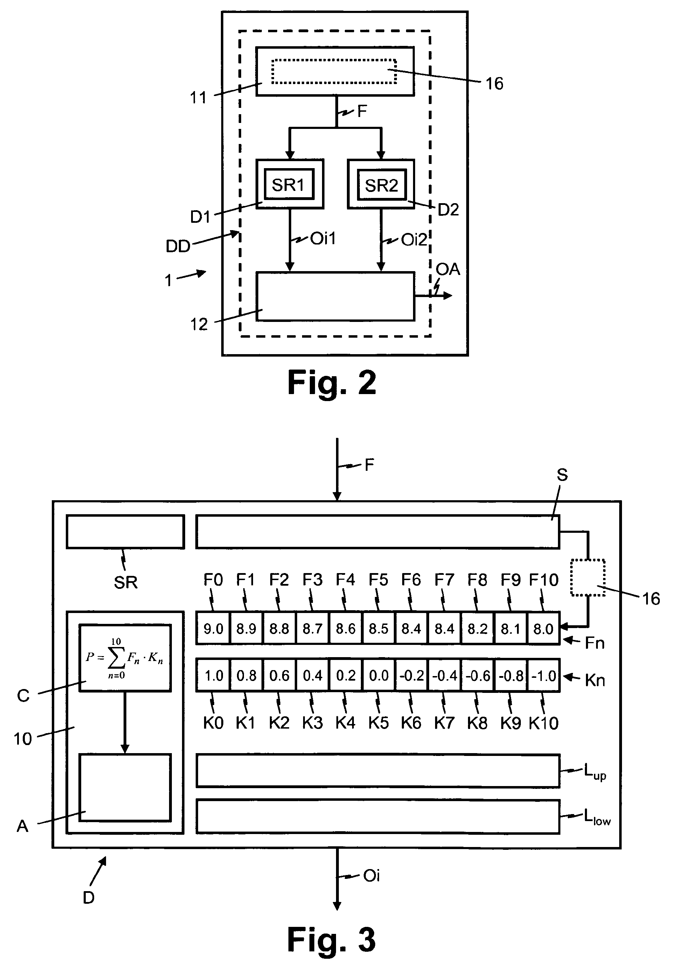

In Figures 1 and 2, reference numeral 1 refers to an injection device for injecting automatically a medicament, e.g. insulin, through a catheter 8 under a patient's skin. Figure 1 shows an example of the structural configuration of the injection device 1. The injection device 1 has a pump mechanism accommodated in a housing 6, a reservoir 2 in which the medicament is stored, and an exchangeable energy supply unit (not shown). The pump unit has a piston 3 which comes to lie in the reservoir 2 and which, via a rod-shaped drive member 4, is driven by an electric motor 5 and toothed wheels 7a and 7b. The electric motor 5 and force transmission elements - toothed wheels 7a and 7b that act on a sleeve-shaped, further drive member 9 meshing via a thread 14 with the drive member 4 - are arranged on a "free-floating" base 13, which acts on a force sensor 11 operating as force measurement unit for determining, as measurement values or force measurements Fn, the injection force F applied for injection. One skilled in the art will understand that in alternative embodiments different arrangements and implementations of the force sensor or force measurement unit 11, respectively, as well as alternative drive arrangements are possible. The injection device 1 further comprises a control unit (not shown) with a controller for the electric motor 5. The control unit comprises a processor as well as data and program memory.

In an embodiment, the injection device 1 further comprises a one-way (unidirectional) pressure valve 15, indicated only schematically in Figure 1, arranged at the injection outlet of the injection device 1. The pressure valve 15 is configured to open and let the medicament pass for injection when a defined opening pressure $P_{valve\_opening}$ is reached, e.g. $P_{valve\_opening}$ = 0.9*bar*.

As illustrated schematically in Figure 2, the injection device 1 comprises an occlusion detection system DD configured to detect occlusions on the basis of the injection force F measured by the force measurement unit 11. For that purpose the occlusion detection system DD comprises several functional modules, including two occlusion detectors D1, D2 configured to take force measurements by sampling in parallel at different measurement rates $SR1$, $SR2$ the injection force F measured by the force measurement unit 11. The measurement rates $SR1$, $SR2$ are sampling rates with different sampling times $TS1 = \dfrac{1}{SR1}$ or $TS2 = \dfrac{1}{SR2}$, respectively.

The occlusion detectors D1, D2 are configured to generate occlusion indicating signals Oi1, Oi2 based on a set of force measurements Fn taken at the respective measurement rates $SR1$, $SR2$. The set of force measurements Fn includes a defined equal number N of force measurements F0-F10, e.g. N= 11, taken by the occlusion detectors D1, D2 during a measurement period $TM1 = (N-1) \cdot \dfrac{1}{SR1}$ or $TM2 = (N-1) \cdot \dfrac{1}{SR2}$, respectively. For example, the sampling times are $TS1$= 3 min and $TS2$ = 30min corresponding to measurement periods of $TM1$= 30 min and $TM2$ = 300 min, respectively. Preferably, the sampling times $TS1$ and $TS2$ are set to correspond to the longest and the shortest time interval between consecutive basal releases that may occur for the device. This time interval may, for example, be fixed to 3 minutes for high delivery rates down to a threshold rate below which the interval is extended up to a maximum value of 30 minutes.

With the alarm generator 12, the occlusion detection system DD comprises a further functional module. The alarm generator 12 is configured to generate an occlusion alarm signal OA, if either or both of the occlusion detectors D1, D2 generate an occlusion indicating signal Oi1, Oi2. Depending on the embodiment, the occlusion alarm signal OA is used to control, e.g. stop, the motor 5, and/or generate a visual and/or audible alarm.

In an embodiment, the occlusion detectors D1, D2 can each be individually activated or deactivated so that the occlusion detection system DD operates with either one or both of the occlusion detectors D 1, D2 in parallel..

In a further embodiment, the occlusion detection system DD further comprises a force adjustment module 16 configured to calculate an adjusted force measurement $F_{adjusted}$ by subtracting from the force measurements $F_{measurement}$ in each case a defined force offset value $F_{adjusted}$ = $F_{measurement}$-$F_{offset}$. As illustrated schematically in Figures 2 and 3, the optional adjustment module 16 is implemented as part of the force measurement unit 11 or the occlusion detectors D1, D2, or as a separate functional module of the occlusion detection system DD arranged between the force measurement unit 11 and one or more occlusion detectors D1, D2. Depending on the embodiment, the force offset value $F_{offset}$ includes a first component $F_{valve\_opening}$ = $P_{valve\_opening} \cdot A_{piston}$ which depends on the pressure valve 15, specifically the opening pressure $P_{valve\_opening}$ of the pressure valve 15 and the piston area $A_{piston}$ applying the injection force; a second component $F_{piston}$ which depends on the friction of the piston; a third component $F_{seal}$ which depends on the friction of a seal arranged in the injection device 1, particularly an X-ring; and/or a fourth component $F_{error}$ representative of an estimated measurement error by the force sensor or force measurement unit 11, respectively, in the force measurement $F_{error}$ =$F_{measurement}$ -$F_{effective}$. For example, considering all four components, the force offset value $F_{offset}$ is calculated as defined by equation (1):

$$F_{offset} = P_{valve\_opening} \cdot A_{piston} + F_{piston} - F_{seal} - F_{error} \quad (1)$$

Table 1 shows examples of minimum, maximum, and nominal values for the friction force $F_{piston}$ of the piston 3, the friction force associated with the seal $F_{seal}$, the force $F_{valve\_opening}$ for opening the pressure valve 15, and the measurement error $F_{error}$ in the force measurement $F_{measurement}$, whereby all sigma values are calculated based on the assumption of a CPK-value of 4 (i.e. assuming a symmetrical distribution of +/- 4 sigma).

Table 1

| Force [N] | Minimum | Maximum | Nominal | Sigma (cpk=1.33) |
|---|---|---|---|---|
| Friction force of piston $F_{piston}$ | 0.00 | 6.00 | 3.00 | 0.75 |
| Friction force of seal $F_{seal}$ | 0.00 | 3.00 | 1.50 | 0.375 |
| Opening force for pressure valve $F_{valve\_opening}$ | 4.70 | 7.39 | 6.05 | 0.34 |
| Measurement error $F_{error}$ | -1.00 | 1.00 | 0.00 | 0.25 |
| **Force offset $F_{offset}$** | **3.80** | **11.30** | **7.55** | **0.94** |

Accordingly, the nominal force offset value to be deducted is 7.55 N. The standard variation sigma based on CPK-value of 4 is 0.94 N for the force offset. In a range for the force offset between 3.8 N and 11.3 N, the valve operates according to specifications.

If subtracting the force offset value $F_{offset}$ from the force measurement $F_{measurement}$ produces a negative result, i.e. if the force offset value $F_{measurement} < F_{offset}$ is greater than force measurement, the force adjustment module 16 is configured to set the adjusted force measurement to zero $F_{adjusted} = 0$.

Depending on the embodiment, the occlusion detectors D 1, D2, the alarm generator 12, and/or the force adjustment module 16 are implemented as programmed software modules comprising computer program code for controlling one or more processors (microcontrollers) of the injection device 1, or fully or partly by way of hardware components. It should be pointed out that the force adjustment module 16 can be implemented in an injection device 1 with an occlusion detection system DD comprising and operating with either one or both of the occlusion detectors D1, D2, or with even further occlusion detectors.

In Figure 3, reference numeral D refers to an embodiment of the occlusion detectors D1, D2. As illustrated in Figure 3, embodiment D of the occlusion detectors D1, D2 comprises several functional modules including a sampling module S and a control module 10 with a computing module C and an analyzing module A. The embodiment D of the occlusion detectors D 1, D2 further comprises a memory unit SR for storing the current measurement (sampling) rate $SR1$, $SR2$, an array of memory units F0-F10 for storing the set of force measurements Fn taken by the sampling module S at the current measurement rates $SR1$, $SR2$, an array of memory units K0-K10 for storing a set of weighting factors Kn, and memory units for storing values of an upper limit $L_{up}$ and a lower limit $L_{low}$.

The computing module C is configured to calculate from the set of force measurements Fn and the vector of weighting factors Kn the scalar or dot product

$$P = \sum_{n=0}^{10} F_n \cdot K_n$$

The analyzing module A is configured to compare the calculated scalar or dot product $P$ to the current values of the upper limit $L_{up}$ and the lower limit $L_{low}$ and to generate an occlusion indicating signal Oi, Oi1, Oi2 depending on the result of that comparison, as will be described below.

Depending on the embodiment, the sampling module S and/or the control module 10 with the computing module C and analyzing module A are implemented as programmed software modules comprising computer program code for controlling one or more processors (microcontrollers) of the injection device 1, or fully or partly by way of hardware components.

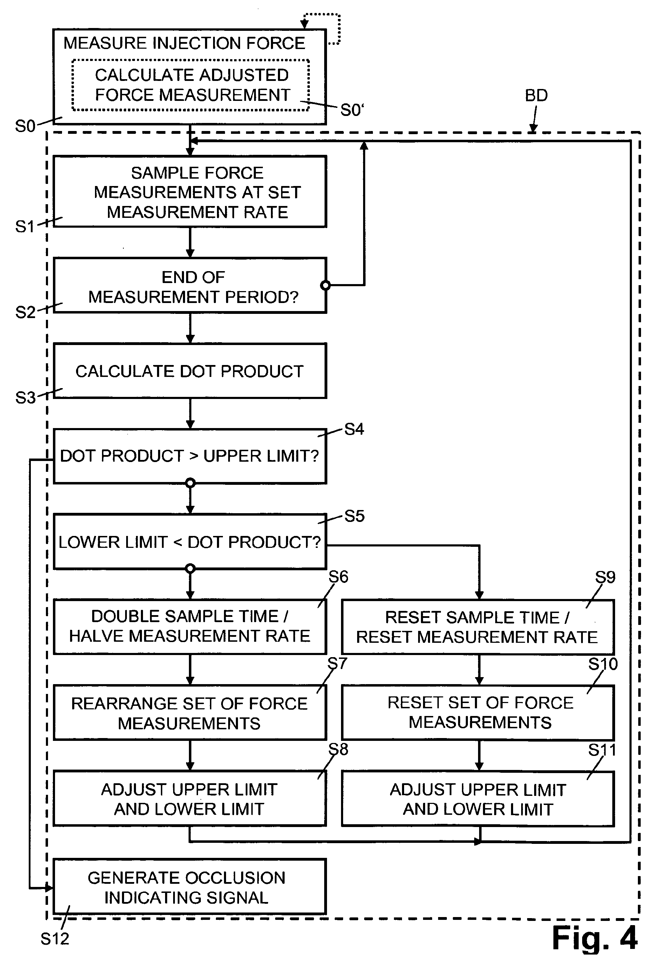

In the following paragraphs, described with reference to Figure 4 are possible sequences of steps performed by the occlusion detection system DD or its functional modules, respectively, for detecting an occlusion in the injection device 1.

In step S0, the injection force F is measured continuously or periodically by the force sensor 11 operating as force measurement unit. Optionally, e.g. if the injection device 1 is provided with a pressure valve 15 at its outlet, in step SO', the force measurement $F_{measurement}$ is adjusted by the adjustment module 16 calculating an adjusted force measurement $F_{adjusted} = F_{measurement} - F_{offset}$, as described above.

In an embodiment, in addition or as an alternative to compensating for the opening pressure $P_{valve\_opening}$ of the valve 15 by subtracting a respective force offset from the force measurement $F_{measurement}$, operation of the occlusion detection system DD or the occlusion detectors D1, D2, respectively, is delayed by a defined time delay $T_{delay}$ after the priming phase. The time delay $T_{delay}$ is implemented by the control module 10 based on a set delay parameter Delay

and ensures that there will be no incorrect alarms after the sniffing phase, if there is no priming phase. The delay parameter *Delay* is expressed in [IU]; this means that the occlusion detection system DD or the occlusion detectors D 1, D2, respectively, are not active after the sniffing phase as long as the amount of IUs, indicated by the delay parameter *Delay* , has not been administered. The control module 10 is configured to determine the time delay $T_{delay}$ based on the set basal rate *BasalRate* , defined in [UI/h] and the delay parameter *Delay* using equation (2), where $T_{StartBasal}$ can be set to zero:

$$T_{delay} = \frac{Delay \cdot 3600}{BasalRate} + T_{StartBasal} \quad (2)$$

In block BD, steps S1-S12 are performed for detecting occlusions in the injection device 1.

In step S1, at the set current measurement rate *SR*1, *SR*2, the occlusion detector D1, D2 takes a force measurement Fn from the force measurement unit 11. Preferably, the force measurements Fn are taken in sync with the injection of the medicament, preferably at a defined duration of time, e.g. one second, before an injection. In other words, the injection force F is sampled in sync with the injection of the medicament. In correspondence with its time or position n in the measurement period *TM*1, *TM*2 the force measurement Fn is stored in the memory units F0-F10. For example, in the embodiment D shown in Figure 3, the first or oldest force measurement of the current measurement period *TM*1, *TM*2 is stored in memory unit F10 ("8.0"), whereas the last or newest force measurement of the current measurement period *TM*1, *TM*2 is stored in memory unit F0 ("9.0").

In step S2, the control module 10 checks whether the end of the current measurement period *TM*1, *TM*2 has been reached, i.e. whether the set of force measurements Fn for the current measurement period *TM*1, *TM*2 is complete and holds the defined number of force measurements, e.g. n=11, taken during the current measurement period *TM*1, *TM*2 at the current measurement rate *SR*1, *SR*2. If the current measurement period *TM*1, *TM*2 has not ended, processing continues in step S1 by taking the next force measurement Fn. Otherwise, if the current measurement period *TM*1, *TM*2 has ended, processing continues in step S3.

In step S3, the computing module C calculates from the current set of force measurements Fn and the vector of weighting factors Kn the scalar or dot product $P = \sum_{n=0}^{10} F_n \cdot K_n$ . Depending on the embodiment, an identical or different set of weighting factors Kn is used for the detectors D1, D2.

In step S4, the analyzing module A checks whether the value of the calculated dot product *P* is greater than the upper limit $P > L_{up}$. If the dot product *P* is greater than the upper limit $P > L_{up}$, processing continues in step S12 with generating an occlusion indicating signal Oi. Otherwise, if the dot product *P* is not greater than the upper limit $P \leq L_{up}$, processing continues in step S5.

In step S5, the analyzing module A checks whether the value of the calculated dot product *P* is lower than the lower limit $P < L_{low}$. If the dot product *P* is lower than the lower limit $P < L_{low}$, processing continues in step S9; otherwise, if the dot product *P* is not lower than the lower limit, i.e. the dot product is within the range of the upper and lower limits $L_{low} \leq P \leq L_{up}$, processing continues in step S6.

In step S6, the control module 10 increases the measurement period *TM*1, *TM*2 for the respective occlusion detector D1, D2 that had a dot product within the value range of upper and lower limits $L_{low} \leq P \leq L_{up}$. Specifically, the respective measurement period *TM*1, *TM*2 and sample time *TS*1, *TS*2 are doubled, and the corresponding measurement rate *SR*1, *SR*2 is halved accordingly. Respective modes "Mode1", "Mode2", "Mode4", "Mode8", and "Mode16" indicate how many times the respective measurement period *TM*1, *TM*2 or sample time *TS*1, *TS*2 have been multiplied, e.g. "Mode 1" indicates that the current measurement period *TM*1, *TM*2 or sample time *TS*1, *TS*2 corresponds to the initial measurement period *TM*1, *TM*2 or sample time *TS*1, *TS*2, respectively; whereas "Mode 8" indicates that the current measurement period *TM*1, *TM*2 or sample time *TS*1, *TS*2 corresponds to an eightfold of the initial measurement period *TM*1, *TM*2 or sample time *TS*1, *TS*2, respectively.

In step S7, the control module 10 rearranges the set of force measurements stored in the memory units F0-F10 in accordance with the extended measurement period *TM*1, *TM*2 and corresponding measurement rate *SR*1, *SR*2. Specifically, kept are those force measurements F0, F2, F4, F6, F8, F10 which were stored for the measurement period *TM*1, *TM*2 before the extension, but were taken in sync with the new measurement rate; while deleted are those force measurements F9, F7, F5, F3, F1 which were not taken in sync with the new measurement rate. Table 2, illustrates how the set of force measurements is rearranged through this adjustment process by showing contents of the memory units F0-F10 for before and after the adjustment or rearrangement process, in the transition from "Mode 1" to "Mode 2", whereby those values that are kept are indicated in bold.

Table 2

| Before (Mode 1) | |
|---|---|
| F10 | **8.0** |
| F9 | 8.1 |
| F8 | **8.2** |
| F7 | 8.4 |
| F6 | **8.4** |
| F5 | 8.5 |
| F4 | **8.6** |
| F3 | 8.7 |
| F2 | **8.8** |
| F1 | 8.9 |
| F0 | **9.0** |
| After (Mode 2) | |
| F10 | **8.0** |
| F9 | **8.2** |
| F8 | **8.4** |
| F7 | **8.6** |
| F6 | **8.8** |
| F5 | **9.0** |
| F4 | 0.0 |
| F3 | 0.0 |
| F2 | 0.0 |
| F1 | 0.0 |
| F0 | 0.0 |

As is illustrated in Table 2, the contents of F10, i.e. the oldest and first force measurement in the set, remains the same, whereas due to the slower measurement rate the contents of F8, F6, F4, F2 and F0 become the contents of F9, F8, F7, F6 or F5, respectively.

Likewise, Table 3 illustrates the adjustment and rearrangement of the set of force measurements stored in the memory units F0-F10 when the initial measurement period $TM1$, $TM2$ ("Mode 1") is doubled ("Mode 2"), quadrupled ("Mode 4") and octuplicated ("Mode 8") in length, respectively.

Table 3

| Initial measurement period (Mode 1) | |
|---|---|
| F10 | **8.0** |
| F9 | 8.1 |
| F8 | **8.2** |
| F7 | 8.4 |
| F6 | **8.4** |
| F5 | 8.5 |
| F4 | **8.6** |

(continued)

| Initial measurement period (Mode 1) | |
| --- | --- |
| F3 | 8.7 |
| F2 | **8.8** |
| F1 | 8.9 |
| F0 | **9.0** |
| Doubled measurement period (Mode 2) | |
| F10 | **8.0** |
| F9 | **8.2** |
| F8 | **8.4** |
| F7 | **8.6** |
| F6 | **8.8** |
| F5 | **9.0** |
| F4 | 0.0 |
| F3 | 0.0 |
| F2 | 0.0 |
| F1 | 0.0 |
| F0 | 0.0 |
| Quadrupled measurement period (Mode 4) | |
| F10 | **8.0** |
| F9 | **8.4** |
| F8 | **8.8** |
| F7 | 0.0 |
| F6 | 0.0 |
| F5 | 0.0 |
| F4 | 0.0 |
| F3 | 0.0 |
| F2 | 0.0 |
| F1 | 0.0 |
| F0 | 0.0 |
| Octuplicated measurement period (Mode 8) | |
| F10 | **8.0** |
| F9 | 0.0 |
| F8 | 0.0 |
| F7 | 0.0 |
| F6 | 0.0 |
| F5 | 0.0 |
| F4 | 0.0 |

(continued)

| Octuplicated measurement period (Mode 8) | |
|---|---|
| F3 | 0.0 |
| F2 | 0.0 |
| F1 | 0.0 |
| F0 | 0.0 |

In step S8, the control module 10 adjusts the values of upper limit $L_{up}$ and the lower limit $L_{low}$ that are applicable to the current length of the measurement period $TM1$, $TM2$. Subsequently, processing continues in step S1 by sampling the injection force F at the new sample time $TS1$, $TS2$ or sampling rate $SR1$, $SR2$, respectively. Table 4 shows examples of defined values for the upper limit $L_{up}$ and the lower limit $L_{low}$ as well as the corresponding sample times $TS1$, $TS2$ for the measurement periods $TM1$, $TM2$ according to Modes 1, 2, 4, 8 and 16, respectively.

Table 4

| Measurement mode | 1 | 2 | 4 | 8 | 16 |
|---|---|---|---|---|---|
| upper limit $L_{up}$ | 8.03 | 7.7 | 7.04 | 5.75 | 4.4 |
| lower limit $L_{low}$ | 0.275 | 0.55 | 1.1 | 2.2 | 4.4 |
| Sample time $TS1$, $TS2$ | 3/30 | 6/60 | 12/120 | 24/240 | 48/480 |
| Measurement period $TM1$, $TM2$ | 30/300 | 60/600 | 120/1200 | 240/2400 | 480/4800 |

In step S9, the control module 10 resets the measurement period $TM1$, $TM2$ for the respective occlusion detector D1, D2 that had a dot product below the lower limit $L_{low}$. Specifically, the respective measurement period $TM1$, $TM2$ and sample time $TS1$, $TS2$, and accordingly the corresponding measurement rate $SAR1$, $SR2$, are reset to their initial values of "Mode 1".

In step S10, the control module 10 resets the set of force measurements Fn stored in the memory units F0-F10 to zero.

In step S11, the control module 10 resets the values of upper limit $L_{up}$ and the lower limit $L_{low}$ to their initial values of "Mode 1 ". Subsequently, processing continues in step S1 by sampling the injection force F at the reset sample time $TS1$, $TS2$ or sampling rate $SR1$, $SR2$, respectively.

In step S12, the control module 10 generates an occlusion indicating signal Oi, Oi1, Oi2 for the respective occlusion detector D 1, D2 that had a dot product above the upper limit $L_{up}$.

Responsive to an occlusion indicating signal Oi, Oi1, Oi2 from either or both of the occlusion detectors D1, D2, the alarm generator 12 generates an occlusion alarm signal OA.

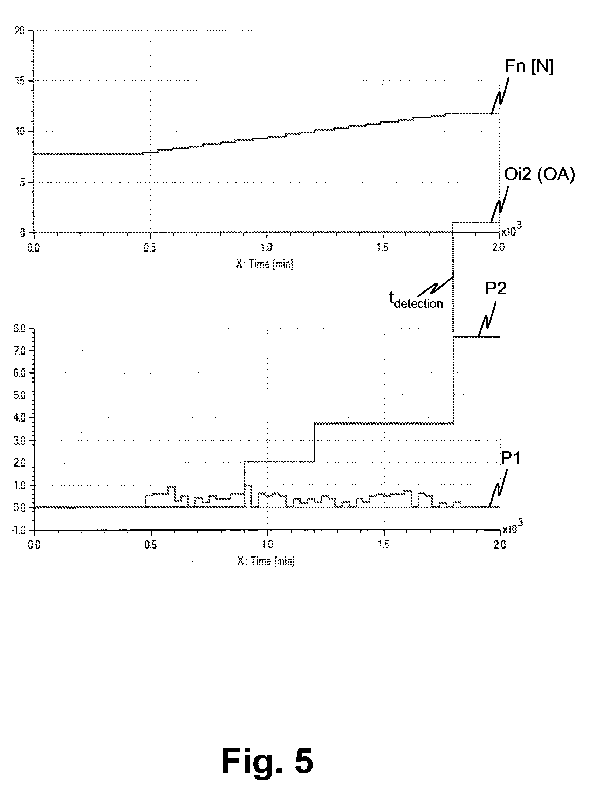

Figure 5 shows an example of occlusion detection by the occlusion detection system DD for a basal delivery rate of 0.02 [IU/h], a basal delivery interval of 2 [h], a priming volume of 0 [IU], and a delay parameter of 0.5 [IU]. The occlusion detector D1 is set to a take force measurements Fn at a measurement rate $SR1$ corresponding to a sampling time $TS1$ of 3 minutes. The occlusion detector D2 is set to a take force measurements Fn at a measurement rate $SR2$ corresponding to a sampling time $TS2$ of 30 minutes.

Specifically, Figure 5 illustrates in the top graph the temporal course of the force measurements $F_n$ in [N] (depending on the embodiment adjusted or not adjusted by the force offset) after a time delay. The top graph further shows the occlusion indicating signal Oi2 generated by the occlusion detector D2. In the bottom graph, Figure 5 illustrates the dot product P1 generated by the occlusion detector D1 operating at the sampling time $TS1$ of 3 minutes and the dot product P2 generated by the occlusion detector D2 operating at the sampling time $TS2$ of 30 minutes. As can be seen in Figure 5, the occlusion detector D1 operating at the sampling time $TS1$ of 3 minutes always stays in the 30 minute evaluation period and is, therefore, not configured to detect an occlusion. However, in the present example, the occlusion detector D2 operating at the sampling time $TS2$ of 30 minutes is configured to detect an occlusion and, thus, generate a corresponding occlusion indicating signal Oi2 at the detection time $t_{detection}$ for which the alarm generator 12 will generate a corresponding occlusion alarm signal OA. For a high delivery rate, in contrast, an occlusion would be detected by occlusion detector D1, but not by occlusion detector D2. The arrangement of more than one occlusion detector allows the detection of occlusions over a large range of basal delivery rates. In addition to the occlusion detectors as described herein, a further occlusion detector with a fixed force threshold value may be present as fallback for situations where the somewhat more sophisticated occlusion detectors fail to correctly detect the presence of an occlusion due to a data artefact or the like.

It should be noted that, in the description, the occlusion detectors D 1, D2 were each associated with an array of eleven array elements for measuring, recording, and processing a set of eleven force measurements Fn over a measurement period $TM1$, $TM2$ including ten times the sample time $TS1$, $TS2$; however, one skilled in the art will understand that the occlusion detectors D 1, D2 and the array can alternatively be configured for different numbers of force measurements Fn and measurement periods $TM1$, $TM2$, e.g. an array with nine or seventeen elements for recording and processing a corresponding number of force measurements Fn over a measurement period $TM1$, $TM2$ including eight or sixteen times the respective sample time $TS1$, $TS2$. Likewise, in the described examples, the initial sampling times $TS1$, $TS2$ are set to correspond to the defined lower delivery rate limit (minimum delivery rate) and the defined upper delivery rate limit (maximum delivery rate) of three and thirty minutes, respectively, but one skilled in the art will understand that different values for the initial sampling times $TS1$, $TS2$ or corresponding minimum and maximum delivery rates are possible. Moreover, the computer program code has been associated with specific functional modules and the sequence of the steps has been presented in a specific order, one skilled in the art will understand, however, that the computer program code may be structured differently and that the order of at least some of the steps could be altered, without deviating from the scope of the invention.

**Claims**

1. An injection device (1) for injecting automatically a medicament, the injection device (1) comprising a force measurement unit (11) configured to measure an injection force (F), wherein the injection device (1) further comprises:

   - a first occlusion detector (D1) configured to take force measurements (Fn) at a first measurement rate (SR1), and to generate a first occlusion indicating signal (Oi1) based on a set of force measurements (Fn) taken at the first measurement rate (SR1);
   - a second occlusion detector (D2) configured to take force measurements (Fn) at a second measurement rate (SR2), lower than the first measurement rate (SR2), and to generate a second occlusion indicating signal (Oi2) based on a set of force measurements (Fn) taken at the second measurement rate (SR2); and
   - an alarm generator (12) configured to generate an occlusion alarm signal (OA) in cases where the first occlusion indicating signal (Oi1) is generated by the first occlusion detector (D1) or the second occlusion indicating signal (Oi2) is generated by the second occlusion detector (D2).

2. The injection device (1) of claim 1, wherein the injection device (1) is configured to inject the medicament at a variable delivery rate, the first measurement rate (SR1) corresponds to a defined upper delivery rate limit, and the second measurement rate (SR2) corresponds to a defined lower delivery rate limit, lower than the defined upper delivery rate limit.

3. The injection device (1) of one of claims 1 or 2, wherein the first occlusion detector (D1) is configured to generate the first occlusion indicating signal (Oi1) based on a set of force measurements (Fn), taken at the first measurement rate (SR1) during a first measurement period; and the second occlusion detector (D2) is configured to generate the second occlusion indicating signal (Oi2) based on a set of force measurements (Fn), taken at the second measurement rate (SR2) during a second measurement period longer than the first measurement period.

4. The injection device (1) of one of claims 1 to 3, wherein the first occlusion detector (D1) is configured to generate the first occlusion indicating signal (Oi1) based on a first calculated product (P1) of a first vector of weighting factors (Kn) and the set of force measurements (Fn), taken at the first measurement rate (SR1) during a first measurement period; and the second occlusion detector (D2) is configured to generate the second occlusion indicating signal (Oi2) based on a second calculated product (P2) of a second vector of weighting factors (Kn) and the set of force measurements (Fn), taken at the second measurement rate (SR2) during a second measurement period longer than the first measurement period.

5. The injection device (1) of claim 4, wherein the first occlusion detector (D1) is configured to halve the first measurement rate (SR1) in case the first calculated product (P1) is within a first defined threshold range; and the second occlusion detector (D2) is configured to halve the second measurement rate (SR2) in case the second calculated product (P2) is within a second defined threshold range.

6. The injection device (1) of one of claims 4 or 5, wherein the first occlusion detector (D1) is configured to extend the first measurement period by doubling its duration in case the first calculated product (P1) is within a first defined threshold range; and the second occlusion detector (D2) is configured to extend the second measurement period by doubling its duration in case the second calculated product (P2) is within a second defined threshold range.

7. The injection device (1) of claim 6, wherein the first occlusion detector (D1) is configured to include in the extended first measurement period force measurements (Fn), taken at the first measurement rate (SR1) before extending the first measurement period; and the second occlusion detector (D2) is configured to include in the extended second measurement period force measurements (Fn), taken at the second measurement rate (SR2) before extending the second measurement period.

8. The injection device (1) of one of claims 4 to 7, wherein the first occlusion detector (D1) is configured to generate the first occlusion indicating signal (Oi1) in case the first calculated product exceeds a first upper threshold value ($L_{up}$); and the second occlusion detector (D2) is configured to generate the second occlusion indicating signal (Oi2) in case the second calculated product exceeds a second upper threshold value ($L_{up}$).

9. The injection device (1) of one of claims 1 to 8, further comprising an occlusion detection system (DD) configured to deduct from the force measurements (Fn) a force offset value, and to detect the occlusions based on the force measurements (Fn) having the force offset value deducted therefrom.

**Patentansprüche**

1. Injektionsgerät (1) zum automatischen Injizieren eines Medikaments, wobei das Injektionsgerät (1) eine Kraftmesseinheit (11) umfasst, die so ausgelegt ist, dass sie eine Injektionskraft (F) misst, wobei das Injektionsgerät (1) des Weiteren umfasst:

   - einen ersten Okklusionsdetektor (D1), der so ausgelegt ist, dass er Kraftmessungen (Fn) mit einer ersten Messrate (SR1) durchführt und ein erstes Okklusionsanzeigesignal (Oi1) auf Basis einer Menge von mit der ersten Messrate (SR1) durchgeführten Kraftmessungen (Fn) erzeugt;
   - einen zweiten Okklusionsdetektor (D2), der so ausgelegt ist, dass er Kraftmessungen (Fn) mit einer zweiten Messrate (SR2) durchführt, die geringer als die erste Messrate (SR2) ist, und ein zweites Okklusionsanzeigesignal (Oi2) auf Basis einer Menge von mit der zweiten Messrate (SR2) durchgeführten Kraftmessungen (Fn) erzeugt;
   - einen Alarmgenerator (12), der so ausgelegt ist, dass er ein Okklusionsalarmsignal (OA) erzeugt, wenn das erste Okklusionsanzeigesignal (Oi1) vom ersten Okklusionsdetektor (D1) erzeugt wird oder das zweite Okklusionsanzeigesignal (Oi2) vom zweiten Okklusionsdetektor (D2) erzeugt wird.

2. Injektionsgerät (1) nach Anspruch 1, wobei das Injektionsgerät (1) so ausgelegt ist, dass es das Medikament in einer variablen Abgaberate injiziert, wobei die erste Messrate (SR1) einem definierten oberen Grenzwert der Abgaberate entspricht und die zweite Messrate (SR2) einem definierten unteren Grenzwert der Abgaberate entspricht, die geringer als der definierte obere Grenzwert der Abgaberate ist.

3. Injektionsgerät (1) nach einem der Ansprüche 1 oder 2, wobei der erste Okklusionsdetektor (D1) so ausgelegt ist, dass er das erste Okklusionsanzeigesignal (Oi1) auf Basis einer Menge von Kraftmessungen (Fn) erzeugt, die mit der ersten Messrate (SR1) während eines ersten Messzeitraums durchgeführt wurden; und der zweite Okklusionsdetektor (D2) so konfiguriert ist, dass er das zweite Okklusionsanzeigesignal (Oi2) auf Basis einer Menge von Messungen (Fn) erzeugt, die mit der zweiten Messrate (SR2) während eines zweiten Messzeitraums durchgeführt wurden, der länger als der erste Messzeitraum ist.

4. Injektionsgerät (1) nach einem der Ansprüche 1 bis 3, wobei der erste Okklusionsdetektor (D1) so ausgelegt ist, dass er das erste Okklusionsanzeigesignal (Oi1) auf Basis eines ersten berechneten Produkts (P1) eines ersten Vektors von Gewichtungsfaktoren (Kn) und der Menge von Kraftmessungen (Fn) erzeugt, die mit der ersten Messrate (SR1) während eines ersten Messzeitraum durchgeführt wurden; und der zweite Okklusionsdetektor (D2) so ausgelegt ist, dass er das zweite Okklusionsanzeigesignal (Oi2) auf Basis eines zweiten berechneten Produkts (P2) eines zweiten Vektors von Gewichtungsfaktoren (Kn) und einer Menge von Messungen (Fn) erzeugt, die mit der zweiten Messrate (SR2) während eines zweiten Messzeitraums durchgeführt wurden, der länger als der erste Messzeitraum ist.

**5.** Injektionsgerät (1) nach Anspruch 4, wobei der erste Okklusionsdetektor (D1) so ausgelegt ist, dass er die erste Messrate (SR1) halbiert, wenn das erste berechnete Produkt (P1) innerhalb eines ersten definierten Schwellenwertbereichs liegt; und der zweite Okklusionsdetektor (D2) so ausgelegt ist, dass er die zweite Messrate (SR2) halbiert, wenn das zweite berechnete Produkt (P2) innerhalb eines zweiten definierten Schwellenwertbereichs liegt.

**6.** Injektionsgerät (1) nach einem der Ansprüche 4 oder 5, wobei der erste Okklusionsdetektor (D1) so ausgelegt ist, dass er den ersten Messzeitraum durch Verdoppeln dessen Dauer verlängert, wenn das erste berechnete Produkt (P1) innerhalb eines ersten definierten Schwellenwertbereichs liegt; und der zweite Okklusionsdetektor (D2) so ausgelegt ist, dass er den zweiten Messzeitraum durch Verdoppeln dessen Dauer verlängert, wenn das zweite berechnete Produkt (P2) innerhalb eines zweiten definierten Schwellenwertbereichs liegt.

**7.** Injektionsgerät (1) nach Anspruch 6, wobei der erste Okklusionsdetektor (D1) so ausgelegt ist, dass er in den verlängerten ersten Messzeitraum Kraftmessungen (Fn) aufnimmt, die mit der ersten Messrate (SR1) durchgeführt wurden, bevor er den ersten Messzeitraum verlängert; und der zweite Okklusionsdetektor (D2) so ausgelegt ist, dass er in den verlängerten zweiten Messzeitraum Kraftmessungen (Fn) aufnimmt, die mit der zweiten Messrate (SR2) durchgeführt wurden, bevor er den zweiten Messzeitraum verlängert.

**8.** Injektionsgerät (1) nach einem der Ansprüche 4 bis 7, wobei der erste Okklusionsdetektor (D1) so ausgelegt ist, dass er das erste Okklusionsanzeigesignal (Oi1) erzeugt, wenn das erste berechnete Produkt einen ersten oberen Schwellenwert ($L_{up}$) überschreitet; und der zweite Okklusionsdetektor (D2) so ausgelegt ist, dass er das zweite Okklusionsanzeigesignal (Oi2) erzeugt, wenn das zweite berechnete Produkt einen zweiten oberen Schwellenwert ($L_{up}$) überschreitet.

**9.** Injektionsgerät (1) nach einem der Ansprüche 1 bis 8, welches des Weiteren ein Okklusionsdetektionssystem (DD) umfasst, das so konfiguriert ist, dass es einen Kraft-Offsetwert von den Kraftmessungen (Fn) ableitet und die Okklusionen auf Basis der Kraftmessungen (Fn) unter Abzug des Kraft-Offsetwerts von diesen detektiert.

## Revendications

**1.** Dispositif d'injection (1) servant à injecter automatiquement un médicament, le dispositif d'injection (1) comprenant une unité de mesure de force (11) conçue pour mesurer une force d'injection (F), le dispositif d'injection (1) comprenant en outre :

- un premier détecteur d'occlusion (D1) conçu pour prendre des mesures de forces (Fn) au niveau à une première cadence de mesurage (SR1) et pour produire un premier signal d'indication d'occlusion (Oi1) en fonction d'un ensemble de mesures de forces (Fn) prises à la première cadence de mesurage (SR1) ;
- un deuxième détecteur d'occlusion (D2) conçu pour prendre des mesures de forces (Fn) au niveau à une deuxième cadence de mesurage (SR2), inférieur à la première cadence de mesurage (SR2), et pour produire un deuxième signal d'indication d'occlusion (Oi2) en fonction d'un ensemble de mesures de forces (Fn) prises à la deuxième cadence de mesurage (SR2) ; et
- un générateur d'alarme (12) conçu pour produire un signal d'alarme d'occlusion (OA) dans les cas où le premier signal d'indication d'occlusion (Oi1) est produit par le premier détecteur d'occlusion (D1) ou bien où le deuxième signal d'indication d'occlusion (Oi2) est produit par le deuxième détecteur d'occlusion (D2).

**2.** Dispositif d'injection (1) selon la revendication 1, le dispositif d'injection (1) étant conçu pour injecter le médicament à un débit variable, la première cadence de mesurage (SR1) correspondant à une limite supérieure définie de débit, et la deuxième cadence de mesurage (SR2) correspondant à une limite inférieure définie de débit, inférieure à la limite supérieure définie de débit.

**3.** Dispositif d'injection (1) selon l'une des revendications 1 ou 2, dans lequel le premier détecteur d'occlusion (D1) est conçu pour produire le premier signal indiquant une occlusion (Oi1) en fonction d'un ensemble de mesures de forces (Fn), prises à la première cadence de mesure (SR1) pendant une première période de mesures ; et où le deuxième détecteur d'occlusion (D2) est conçu pour produire le deuxième signal indiquant une occlusion (Oi2) en fonction d'un ensemble de mesures de forces (Fn), prises à la deuxième cadence de mesures (SR2) pendant une deuxième période de mesures plus longue que la première période de mesures.

**4.** Dispositif d'injection (1) selon l'une des revendications 1 à 3, dans lequel le premier détecteur d'occlusion (D1) est conçu pour produire le premier signal indiquant une occlusion (Oi1) en fonction d'un premier produit calculé (P1) d'un premier vecteur de facteurs pondérants (Kn) et de l'ensemble de mesures de forces (Fn), prises à la première cadence de mesure (SR1) pendant une première période de mesures ; et où le deuxième détecteur d'occlusion (D2) est conçu pour produire le deuxième signal indiquant une occlusion (Oi2) en fonction d'un deuxième produit calculé (P2) d'un deuxième vecteur de facteurs pondérants (Kn) et de l'ensemble de mesures de forces (Fn), prises à la deuxième cadence de mesures (SR2) pendant une deuxième période de mesures plus longue que la première période de mesures.

5. Dispositif d'injection (1) selon la revendication 4, dans lequel le premier détecteur d'occlusion (D1) est conçu pour réduire de moitié la première cadence de mesure (SR1) si le premier produit calculé (P1) se trouve dans un premier intervalle seuil défini ; et où le deuxième détecteur d'occlusion (D2) est conçu pour réduire de moitié la deuxième cadence de mesures (SR2) si le deuxième produit calculé (P2) se trouve dans un deuxième intervalle seuil défini.

6. Dispositif d'injection (1) selon la revendication 4 ou 5, dans lequel le premier détecteur d'occlusion (D1) est conçu pour prolonger la première cadence de mesure en doublant sa durée si le premier produit calculé (P1) se trouve dans un premier intervalle seuil défini ; et où le deuxième détecteur d'occlusion (D2) est conçu pour prolonger la deuxième cadence de mesures si le deuxième produit calculé (P2) se trouve dans un deuxième intervalle seuil défini.

7. Dispositif d'injection (1) selon la revendication 6, dans lequel le premier détecteur d'occlusion (D1) est conçu pour comprendre, pendant la première période de mesures prolongée, mesures de forces (Fn), prises à la première cadence de mesure (SR1) avant l'extension de la première période de mesures; et où le deuxième détecteur d'occlusion (D2) est conçu pour produire, pendant la deuxième période de mesures prolongée, des mesures de forces (Fn), prises à la deuxième cadence de mesures (SR2) avant la prolongation de la deuxième période de mesures.

8. Dispositif d'injection (1) selon l'une des revendications 4 à 7, dans lequel le premier détecteur d'occlusion (D1) est conçu pour produire le premier signal indiquant une occlusion (Oi1) si le premier produit calculé dépasse une première valeur seuil ($L_{up}$) ; et où le deuxième détecteur d'occlusion (D2) est conçu pour produire le deuxième signal indiquant une occlusion (Oi2) si le deuxième produit calculé dépasse une deuxième valeur seuil ($L_{up}$).

9. Dispositif d'injection (1) selon l'une des revendications 1 à 8, comprenant en outre un système de détection d'occlusion (DD) conçu pour déduire des mesures de force (Fn) une valeur décalée de force, et pour détecter les occlusions en fonction des mesures de force (Fn) dont la valeur décalée de force est déduite de celles-ci.

EP 2 729 200 B1

**Fig. 1**

15

# Fig. 2

$$P = \sum_{n=0}^{10} F_n \cdot K_n$$

| F0 | F1 | F2 | F3 | F4 | F5 | F6 | F7 | F8 | F9 | F10 |
|-----|-----|-----|-----|-----|-----|-----|-----|-----|-----|-----|
| 9.0 | 8.9 | 8.8 | 8.7 | 8.6 | 8.5 | 8.4 | 8.4 | 8.2 | 8.1 | 8.0 |

| 1.0 | 0.8 | 0.6 | 0.4 | 0.2 | 0.0 | -0.2 | -0.4 | -0.6 | -0.8 | -1.0 |
|-----|-----|-----|-----|-----|-----|-----|-----|-----|-----|-----|
| K0 | K1 | K2 | K3 | K4 | K5 | K6 | K7 | K8 | K9 | K10 |

# Fig. 3

MEASURE INJECTION FORCE

CALCULATE ADJUSTED
FORCE MEASUREMENT — S0'

S0

BD

SAMPLE FORCE
MEASUREMENTS AT SET
MEASUREMENT RATE

S1

END OF
MEASUREMENT PERIOD?

S2

CALCULATE DOT PRODUCT

S3

S4
DOT PRODUCT > UPPER LIMIT?

S5
LOWER LIMIT < DOT PRODUCT?

S6
DOUBLE SAMPLE TIME /
HALVE MEASUREMENT RATE

S9
RESET SAMPLE TIME /
RESET MEASUREMENT RATE

S7
REARRANGE SET OF FORCE
MEASUREMENTS

S10
RESET SET OF FORCE
MEASUREMENTS

S8
ADJUST UPPER LIMIT
AND LOWER LIMIT

S11
ADJUST UPPER LIMIT
AND LOWER LIMIT

GENERATE OCCLUSION
INDICATING SIGNAL

S12

**Fig. 4**

**Fig. 5**

18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6659980 B
- WO 200953032 A
- US 5989222 A
- EP 1529546 A1
- US 2005234382 A1
- WO 2008106108 A1